# EUROPEAN PATENT APPLICATION

(11) **EP 1 990 424 A1**
(43) Date of publication of application: **12.11.2008**
(21) Application number: 07290569.8
(22) Date of filing: 07.05.2007
(51) Int. Cl.: C12Q 1/68, G01N 33/68

(54) **Polymorphic length of foxe 1 alanine stretch and genetic susceptibility to thyroid dysgenesis**

(71) Applicant: INSTITUT NATIONAL DE LA SANTE ET DE LA RECHERCHE MEDICALE (INSERM), 75013 Paris (FR)
(72) Inventor: The designation of the inventor has not yet been filed
(74) Representative: Ahner, Francis

(57) **Abstract**

The present invention concerns a method for diagnosis an increased likelihood of developing a thyroid dysgenesis (TD) for an individual, wherein said method comprises determining the length of the polyalanine repeat of the protein encoded by at least one allele of the FOXE1 gene in a tissue sample obtained from said individual; a kit comprising at least one nucleic acid probe or oligonucleotide which can be used in such a method; and a use of such a kit for the diagnosis of an increased likelihood of developing a thyroid dysgenesis (TD) in an individual.

## Description

### Background of the Invention

### Field of the invention

The invention relates to thyroid dysgenesis (TD), and more specifically to a method for diagnosis an increased likelihood of developing a thyroid dysgenesis (TD) based on specific polymorphisms of the FOXE1 gene in a tissue sample obtained from an individual.

### Description of prior art

Abnormalities in thyroid development (Thyroid dysgenesis (TD)) are the main causes of congenital hypothyroidism (CH), which is the most frequent congenital endocrine disorder, affecting one newborn in 3500 in France (CASTANET et al., J. Clin. Endocrinol. Metab., vol.86, p:2009-2014, 2001). TD includes in order of decreasing frequency: 1) incomplete downward migration of the median thyroid anlage during embryonic development resulting in an ectopic sublingual thyroid; 2) defects in the differentiation or survival of thyroid follicular cells resulting in thyroid agenesis or athyreosis; 3) hypoplasia of a thyroid that is otherwise of the normal shape and in the normal pretracheal position ; and 4) thyroid hemiagenesis (VAN VLIET, The Thyroid : A Fundamental and Clinical Text., Lippincott Williams & Wilkins, New York, p:1029-1047, 2005).

Actually, the newborns suffering from congenital hypothyroidism (CH) resulting from thyroid dysgenesis (TD) are systematically screened and treated during the neonatal period by a thyroid hormones supplementation enabling a normal development in most of the cases. Nevertheless, it still exists neurologic abnormalities for some newborns despite this supplementation. It is envisaged that these abnormalities may result from an insufficient increase of thyroid hormone synthesis by the thyroid's mother during pregnancy and therefore to insufficient T4 transfer from the mother to the foetus. It must be noted that maternal T4 is crucial in the neuropsychological development of the foetus.

Although TD is predominantly sporadic, a familial component of TD has been described in 2% of children with CH from TD (15-fold higher than expected by chance alone) strongly suggesting a genetic component (CASTANET *et al.,* abovementioned 2001; CASTANET et al., N. Engl. J. Med., vol.343, p:441-442, 2000) To date, five genes (*FOXE1, PAX8, TSHR, TTF1* and *NKX2.5*) have been involved in thyroid development (MANSOURI et al.. Nat. Genet., vol.19(1), p:87-90, 1998; DE FELICE et al,. Nat. Genet., vo1.19, p:395-8, 1998; SURA-TRUEBA et al.,. J. Clin. Endocrinol. Metab., vol.90, p:455-462, 2005) and four of them (i.e., *FOXE1, PAX8, TTF1* and *TSHR)* are also involved in thyroid hormones synthesis due, for three of those (i.e., *FOXE1, PAX8,* and *TTF1),* to their role in thyroglobulin and thyroid peroxidase genes transactivation, which genes are critical for said thyroid hormones synthesis. In humans, germline mutations in these genes have been identified in patients affected by TD but account for only a small proportion of patients (DE FELICE and DI LAURO, Endocr. Rev., vol.25, p:722-46, 2004; CLIFTON-BLIGH et al., Nat. Genet., vol.19, p:399-401, 1998; VILAIN et al., J. Clin. Endocrinol. Metab., vol.86, p:234-8, 2001; DEVRIENDT et al.,. N. Engl. J. Med., vol.338(18), p:1317-8, 1998; DE ROUX et al., J. Clin. Endocrinol. Metab., vol.81(12), p:4229-35, 1996; DENTICE et al., J. Clin. Endocrinol. Metab., vol.91(4), p:1428-33, 2006). Moreover, linkage studies in some multiplex families have demonstrated that other genes could be involved in TD (CASTANET et al., Eur. J. Hum. Genet., vo1.13, p:232-239, 2005).

More specifically, *FOXE1* encodes specifically a transcription factor that contains a forkhead domain and a polyalanine (polyA) stretch of variable length. Homozygous null mice embryos with targeted disruption of *FOXE1,* exhibit cleft palate and thyroid malformation consisting of either thyroid agenesis or thyroid ectopy, suggesting a role for *Foxe1* in migration as well as in proliferation of the thyroid gland (DE *FELICE et al,.* abovementioned, 1998; PARLATO et al., Dev. Biol., vol.276(2), p:464-75, 2004). In humans, only three different homozygous missense mutations within the forkhead domain have been reported so far to cause athyreosis associated with other malfomations such as cleft palate, bifid epiglottis and choanal atresia (CLIFTON-BLIGH *et al.,* abovementioned, 1998; CASTANET et al., Hum. Mol. Genet., vol.11, p:2051-2059, 2002; BARIS et al., J. Clin. Endocrinol. Metab., vol.91(10), p:4183-7, 2006).

Recently, expansions of trinucleotide repeats encoding polyA tracts have been recognized as the cause of at least nine diseases through the alanine containing proteins (BROWN and BROWN, Trends Genet., vol.20, p:51, 2004). With the exception of PABPN1, which codes for a poly(A)-binding protein, the nine genes with alanine tract expansions described so far encode transcription factors that play important roles during development. Thus, all these polyA disorders result in early developmental effects, such as malformations of the brain or digits (ABU-BAKER and ROULEAU, Biochim. Biophys. Acta., vol.1772(2), p:173-85, 2007). Recently it has been shown that expansions of polyA tracts could result in protein misfolding and aggregation, which is toxic for the cells (CABURET et al., J. Med. Genet., vol.41(12), p:932-6, 2004; ALBRECHT and MUNDLOS, Curr. Opin. Genet. Dev., vol.15, p:285-93, 2005; TROCHET et al., Hum. Mol. Genet., vol.14, p:3697-708, 2005). Moreover, alanine tracts of some transcription factors have been suggested to be involved in the ability to transactivate or repress the expression of target genes (CHADWICK et al., Genomics, vol.41, p:390-396, 1997; CIVITAREALE et al.,. Biochem. J., vol.304, p:981-985, 1994; LAVOIE et al., Hum. Mol. Genet., vol.12, p:2967-79, 2003).

Actually, it still exists a need for identifying a likelihood of developing a thyroid dysgenesis in a subject, more specifically a likelihood of a foetus of developing a thyroid dysgenesis.

### Summary of the invention

One aim of the invention is to provide a tool for diagnosis an increased likelihood of developing a thyroid dysgenesis (TD) for an individual.

In accordance with the present invention there is provided a method for diagnosis an increased likelihood of developing a thyroid dysgenesis (TD), wherein said method comprises determining the length of the polyalanine repeat of the protein encoded by at least one allele of the FOXE1 gene in a tissue sample obtained from an individual.

Preferably, said individual is a female and said method is for diagnosis an increased likelihood of neuropsychological development retardation and/or of developing a thyroid dysgenesis for the foetus during the pregnancy of said female.

In accordance with the present invention there is also provided a kit comprising at least one nucleic acid probe or oligonucleotide which can be used in a method as defined previously for determining the length of the polyalanine repeat of the protein encoded by at least one allele of the FOXE1 gene in a tissue sample obtained from an individual.

In accordance with the present invention there is finally provided the use of the kit as defined previously for the diagnosis of an increased likelihood of developing a thyroid dysgenesis (TD) in an individual.

### Brief Description of the Drawings

The figure 1 shows the frequency of genotypes 14/14, 14/16, and 16/16 in control group and in the both athyreosis and ectopy groups of patients.

The figure 2 shows the effects of FOXE1 coexpressed with PAX8 and TTF1 on transcriptional activity depending of the length of its alanine tract (i.e., 14 or 16 alanines).

The figure 3 shows the expression of FOXE1, PAX8, and TTF1 proteins in 293A transfected cells assessed by Western blot.

### Description of the Preferred embodiments

The inventors have now established that the allele of the transcription factor FOXE1 comprising a polyalanine repeat with 16 successive alanines results is a better enhancer, resulting in an increased transcriptional activity, *vs.* the allele comprising 14 successive alanines.

Moreover, the inventors have established that the allele of FOXE1 comprising a polyalanine repeat with 16 successive alanines confers protection towards the occurrence of TD and in a more marked way for ectopy *νs*. the allele comprising 14 successive alanines tract.

Finally, it is envisaged that a polymorphism in FOXE1 (i.e., polyalanine repeat with 16 successive alanines *vs.* 14 successive alanines), which FOXE1 gene is implicated in thyroid hormones synthesis, is correlated with the thyroid hormones and TSH levels in pregnant women, and potentially with the neuropsychological development retardation and/or with the development of congenital hypothyroidism (CH) resulting from thyroid dysgenesis (TD) in the foetus from these women due to an insufficient thyroid hormone and TSH synthesis by the mother during pregnancy.

Polymorphism of the polyalanine repeat of FOXE1 was reported for the first time by MACCHIA *et al.* with variable length from 12 to 17 alanines (MACCHIA et al., Biochimie. Vol.81(5), p:433-40, 1999). To date, three groups investigated the polyA tract length in TD populations and found polymorphism of this tract with at least 4 different lengths (11, 12, 14, 16 alanines) (HISHINUMA et al., Eur. J. Endocrinol., vol.145, p:385-9, 2001; TONACCHERA et al., Thyroid., vol.14, p:584-8, 2004; SANTARPIA et al., J. Endocrinol. Invest., vol.30(1), p:13-9, 2007). Nevertheless, none of these groups performed the most rigorous analysis that is TDT study in addition to a case-control association study, and the Japanese group performed expression studies for tracts ≤14 without finding any differences in terms of transcriptional activities, suggesting that the polymorphism of the polyA tract was not a cause of developmental defects of the human thyroid gland.

Thus, a first object of the present invention concerns a method for diagnosis an increased likelihood of developing a thyroid dysgenesis (TD), wherein said method comprises determining the length of the polyalanine repeat of the protein encoded by at least one allele of the FOXE1 gene in a tissue sample obtained from an individual.

As used herein, the term "thyroid dysgenesis" comprises any abnormality of thyroid development, such as ectopic sublingual thyroid, athyreosis, and thyroid hemiagenesis.

Preferably, the thyroid dysgenesis corresponds to ectopic sublingual thyroid.

As used herein, the term "polyalanine repeat" refers to at least ten successive alanine amino acids, preferably 11, 12, 14, 16, 17, or 19 successive alanines amino acids.

The methods of diagnosis of the invention preferably refer to *in vitro* methods of diagnosis, wherein a tissue sample is used, which sample has been removed from the body of an individual prior to executing the method of the invention.

In the present invention, a tissue sample comprises preferably cells, such as blood cells, in particular blood platelets, red and/or white blood cells, smooth muscle cells, striated muscle cells, epithelial cells of any epithelium, connective tissue cells of any connective tissue, neurons, tissue samples of the skin, mucosal tissue samples, tissue samples of any organ, and any body fluids, in particular amniotic fluid, whole blood or cord blood (e.g. foetal cord blood), or any blood fraction, liquor, lymph, urine, saliva, and semen.

In the present invention, an individual comprises any vertebrate animal, preferably any mammal, preferably a human, of any age or sex, in particular a foetus, new-born, child, adolescent, adult, or senescent human or animal, any human or animal germ-line cell, a human or animal oocyte or spermatocyte, a human or animal fertilized oocyte, any human or animal being prior to birth, in particular any human or animal embryo or foetus.

In a preferred embodiment of the method of the invention, the protein encoded by at least one allele of the human FOXE 1 gene has the sequence SEQ ID NO:1.

In another embodiment of the method of the invention, the protein encoded by at least one allele of the human FOXE 1 gene is encoded by the sequence SEQ ID NO:2.

In still another embodiment of the method of the invention, the presence in a tissue sample of at least one allele of the FOXE1 gene coding for a protein comprising a polyalanine repeat with 16 successive alanine amino acids is indicative of a reduced risk of thyroid dysgenesis, in particular of a reduced risk of ectopic sublingual thyroid.

In a further embodiment of the method of the invention, the presence in a tissue sample of two alleles of the FOXE1 gene coding for a protein comprising a polyalanine repeat with 14 successive alanine amino acids is indicative of an increased risk of thyroid dysgenesis, in particular of an increased risk of ectopic sublingual thyroid.

In a particular embodiment, the individual is a female, and more preferably a pregnant female.

Advantageously, the method of the invention is for diagnosis an increased likelihood of neuropsychological development retardation and/or of developing a thyroid dysgenesis for the foetus during the pregnancy of said female.

In fact, in view of the results obtained by the inventors, it is envisaged that a polymorphism in FOXE1, which gene is implicated in thyroid hormones synthesis, is correlated with the thyroid hormones and TSH levels in pregnant women, and potentially with the neuropsychological development retardation and/or with the development of congenital hypothyroidism (CH) resulting from thyroid dysgenesis (TD) in the foetus from these women due to an insufficient thyroid hormone and TSH synthesis by the mother during pregnancy.

It has been established that in the prior art that hypothyroidism in pregnant women, which hypothyroidism corresponds to an insufficient thyroid hormone synthesis by said women, may affect their foetuses notably by mental retardation (HADDOW et al., New England Journal of Medicine, vol.341 (8), p:549-555, 1999).

Consequently, the presence in a tissue sample of said female of two alleles of the FOXE1 gene coding for a protein comprising a polyalanine repeat with 14 successive alanine amino acids is indicative of an increased risk of neuropsychological development retardation and/or of developing thyroid dysgenesis for the foetus during pregnancy of said female.

Moreover, the presence in a tissue sample of said female of one allele of the FOXE1 gene coding for a protein comprising a polyalanine repeat with 16 successive alanine amino acids is indicative of a decreased risk of neuropsychological development retardation and/or of developing thyroid dysgenesis for the foetus during pregnancy of said female.

Advantageously, the method of the invention further comprises the step of treating with thyroid hormones supplementation during pregnancy the female for which an increased likelihood of developing a thyroid dysgenesis (TD) has been identified, and said method corresponds to a method for treating and/or preventing a neuropsychological development retardation and/or a thyroid dysgenesis for the foetus of said female.

In some of the above methods, the FOXE1 gene or gene product such as the protein encoded by the FOXE1 gene can be separated and the size of the FOXE1 gene or gene product such as the protein encoded by the FOXE1 gene can be determined by comparison against a known standard.

Examples of separation techniques include chromatography and mass spectrometry techniques. Any chromatographic method that separates the gene or protein from other components in the sample can be used in the present invention. Nonlimiting examples of chromatographic methods include HPLC, dHPLC and the like. The separation can also be performed by electrophoresis.

In some of the above methods, the length of the polyalanine repeat of the protein encoded by at least one allele of the FOXE1 gene can be determined by any method for the sequence analysis of nucleic acids.

In particular, any DNA sequencing protocol based on the DNA sequencing protocol according to Sanger can be used (Current Protocols in Molecular Biology, edited by Fred M. Ausubel, Roger Brent, Robert E. Kingston, David D. Moore, J.G. Seidman, John A. Smith, Kevin Struhl; Looseleaf: 0-471-650338-X; CD-ROM: 0-471-30661-4), in particular using radioactively labeled nucleotides or using nucleotides labeled with a fluorescent dye, in particular involving a polymerase chain reaction (PCR), or using a chemical sequencing method (Pyrosequencing: an accurate detection platform for single nucleotide polymorphisms, Hum Mutat. 2002 May; 19(5):479-85), in particular using pyrosequencing (Pyrosequencing for SNP genotyping, Methods Mol Biol. 2003; 212:189-95; Comparison of GenFlex Tag array and Pyrosequencing in SNP genotyping, J Mol Diagn. 2003 Nov; 5(4):243-9; Microarrays and genetic epidemiology: a multipurpose tool for a multifaceted field. Genet Epidemiol. 2002 Jun; 23(1):4-20, review), or using mass spectrometry for the analysis of a nucleic acid sequence (A novel MALDI-TOF based methodology for genotyping single nucleotide polymorphisms, Nucleic Acids Res. 2003 Dec 15; 31(24):e155; Digital genotyping using molecular affinity and mass spectrometry, Nat Rev Genet. 2003 Dec; 4(12):1001-8).

Preferably, the length of the polyalanine repeat of the protein encoded by at least one allele of the FOXE1 gene can be determined with a DNA sequencing protocol or with a method involving a polymerase chain reaction using at least one oligonucleotide complementary to the sequence of FOXE1 gene or to the sequence of FOXE1 mRNA, preferably at least one oligonucleotide selected in the group comprising SEQ ID NO:3 and SEQ ID NO:4.

It will be understood to those skilled in the art that the present invention readily lends itself to automation. As an automated process, several samples containing the same, or different, combinations of nucleic acids and/or proteins can be assayed in parallel.

According to a further aspect of the present invention, the variations in the length of the polyalanine repeat of the protein encoded by at least one allele of the FOXE1 gene allow to provide a kit for the convenient determination of a predisposition to develop a thyroid dysgenesis.

Thus, a further aspect of the present invention refers to a kit comprising at least one nucleic acid probe or oligonucleotide which can be used in a method as defined in previously for determining the length of the polyalanine repeat of the protein encoded by at least one allele of the FOXE1 gene in a tissue sample obtained from an individual.

Preferably, the oligonucleotide is at least one PCR primer, preferably a set of PCR primers is provided, which allows to amplify a FOXE1 gene fragment comprising the sequence encoding for polyalanine repeat. The skilled person readily provides such an oligonucleotide or set of PCR primers (Current Protocols in Molecular Biology; edited by Fred M. Ausubel et al., supra).

In a preferred embodiment, the kit comprises at least one PCR primer selected in the group comprising SEQ ID NO:3 and SEQ ID NO:4 for determining the length of the polyalanine repeat of the protein encoded by at least one allele of the FOXE1 gene in a tissue sample obtained from an individual.

In one embodiment, the kit is made up of instructions for carrying out any of the methods described herein. The instructions can be provided in any intelligible form through a tangible medium, such as printed on paper, computer readable media, or the like.

The present kits can also include one or more reagents, buffers, hybridization media, nucleic acids, primers, nucleotides, probes, molecular weight markers, enzymes, solid supports, databases, computer programs for calculating dispensation orders and/or disposable lab equipment, such as multi-well plates, in order to readily facilitate implementation of the present methods. Enzymes that can be included in the present kits include nucleotide polymerases and the like. Solid supports can include beads and the like whereas molecular weight markers can include conjugatable markers, for example biotin and streptavidin or the like.

Still a further aspect of the present invention refers to the use of the abovementioned kit comprising at least one nucleic acid probe or oligonucleotide for the diagnosis of an increased likelihood of developing a thyroid dysgenesis (TD).

Advantageously, thyroid dysgenesis is selected in the group comprising ectopic sublingual thyroid, athyreosis, and thyroid hemiagenesis, preferably ectopic sublingual thyroid.

In the following, the invention is described in more detail with reference to amino acid sequences, nucleic acid sequences and the examples. Yet, no limitation of the invention is intended by the details of the examples. Rather, the invention pertains to any embodiment which comprises details which are not explicitly mentioned in the examples herein, but which the skilled person finds without undue effort.

### Example 1: FOXE1 polyalanine repeat length and thyroid dysgenesis occurence

### 1) Materials and Methods

### Subjects and samples

115 unrelated patients, which are all French Caucasian affected with TD were included in the study. Among them, 64 (55.7%) had ectopy, 35 (30.4%) athyreosis and 16 (13.9%) hypoplastic thyroid gland or hemiagenesis.

For the case-control association study, 129 controls of French Caucasian background without history of thyroid disease were used. The sex ratio in both groups was not different (67.0% of women in cases (n=77) *vs*. 57.4% in controls (n=74) (χ² =2.4, *P* = 0.124)).

For the transmission disequilibrium test (TDT), parents of the affected cases were included when possible. A total of 39 trios have been analysed.

The study fulfilled the ethical legislation applied in France, and parental consent was given on behalf of children.

Genomic DNA was extracted from peripheral blood using standard procedures.

### Alanine tract sequencing

Genomic DNA was amplified by PCR (GC rich Taq polymerase, ROCHE DIAGNOSTICS). The following primers were used: FOXE1 F4 5'-GCGGAGGACATGTTCGAGA-3' (SEQ ID NO: 3) and FOXE1 R4 5'-CGCGGGGTAGTAGACTGGAG-3' (SEQ ID NO: 4). The amplicons (260 base pairs) were purified, directly sequenced with the "Big Dye Terminator vl.1 Cycle Sequencing" kit (APPLIED BIOSYSTEMS), in an ABI Prism 3130 automatic sequencer (APPLIED BIOSYSTEMS).

The rest of the *FOXE1* gene was studied by Single Strand Conformation Polymorphism in the patients according to our published protocol (CASTANET *et al., abovementioned,* 2002) and no abnormal migration profile, suggestive of a possible mutation was found.

### Statistical analysis

Data were analysed using the Epi-Info software (version 6.04) and SAS software (version 9.1-SAS INSTITUTE, CARY, NC, USA).

For the case-control study, the allelic and genotypic frequencies of the alanine tract were determined among the cases and controls, and compared with the χ² test. The Hardy-Weinberg Equilibrium (HWE) was tested in the control group and no significant deviation was observed. The strength of the association between each genotype and the disease was estimated by calculating the odds ratio (OR) with 95% confidence interval (95% CI). The genotype 14/14, being the most frequent both in cases and controls, was taken as reference for the calculations. We also tested the strength of the association between our marker and TD according to the gender and to the etiological type of TD.

To validate the case-control association study, a transmission disequilibrium test (TDT) was performed. The TDT enables to test the linkage between the marker locus and a disease susceptibility locus when an association has been found by a case-control study. This test is based on triads (affected child + parents) in which at least one parent being heterozygous for the marker and enable to determine if the allele of interest has been more frequently transmitted by the parents to their affected child (SPIELMAN et al., Am. J. Hum. Genet., vol.52, p:506-16, 1993).

### Plasmid and construction

For the functional studies, we used the FOXE1 expression vector (pFlagCMV-hFOXE1) described previously (CLIFTON-BLIGH *et al.,* abovementioned, 1998) which encodes 14 alanines (given by K. CHATTERJEE). To compare the functional consequences of the 14 and 16 containing alanines tracts, 2 additional alanines were introduced by site-directed mutagenesis (QuickChange Site-directed Mutagenesis Kit, Stratagene) using sense primer 5'-CGCCGCCGCCGCAGCAATCTTCCC-3' (SEQ ID NO: 5) and antisense primer 5'-GGGAAGATTGCTGCGGCGGCGGCG-3' (SEQ ID NO: 6) according to the manufacturer's protocol. The construct was validated by DNA sequencing.

### Transient transfection

For functional studies, the 293A cells lines were grown in DMEM supplemented with 10% fetal bovine serum (BIOWEST) and 1% penicillin and streptomycin (INVITROGEN).

*For luciferase and galactosidase assays,* a reporter gene containing FOXE1, TTF1 and PAX8 binding sites for the thyroid thyroglobulin promoter (given by S. REFETOFF) upstream luciferase was used. We plated the 293A cells at a density 2.10⁵ per well in 24-well 24h before transfection. We then transiently co-transfected cells with 520 ng of reporter gene, 260 ng of pFlagCMV-hFOXE1 (encoding 14 or 16 alanines), 130 ng of pcDNA3-hPAX8 (given by G. Vassart), 130 ng of pcDNA3-hTTF1 (from the pSG5-hTTF1 given by S. REFETOFF) and 150 ng of plasmid BosβGalactosidase (given by K. CHATTERJEE). Co-transfection was carried out by a 6 hour exposure to 3µL of LIPOFECTAMINE in Opti-Mem medium (INVITROGEN). We chose to co-transfect this combination of transcription factors to reproduce physiological conditions, as their simultaneous expression is unique to thyroid follicular cells (SURA-TRUEBA *et al.,.* abovementioned, 2005).

*For Western blot,* 5.10⁶ cells were plated in a 100-mm diameter culture dish 24h before transfection. Cells were then co-transfected with 4µg pcDNA3-hPAXB, 4µg pcDNA3-hTTF1 and 4µg pcDNA3-hFOXE1, using 30µL LIPOFECTAMINE.

*For immunocytochemistry,* 4.10⁴ cells per well were plated in Lab-tek chamber slides (NUNC) 24h before transfection. Cells were then transfected with 400ng of pFlagCMV-hFOXE1 (encoding 14 or 16 alanines) or of pCAGGS-PHOX2B-IRES-EGFP (encoding 20 or 33 alanines given by S. LYONNET) using 1.5µL LIPOFECTAMINE.

### Luciferase and galactosidase assays

For luciferase assays, the transiently co-transfected 293A cells were harvested after 48 hours and luciferase and beta-galactosidase assays were performed. Luciferase values were normalized to beta-galactosidase activity from the internal control plasmid BosβGalactosidase (COLLINGWOOD et al., Mol. Endocrinol., vol.8, p:1262-77, 1994) and represent the mean ± SD of 7 independent experiments each performed in triplicate.

### Western-Blotting

For Western blotting, cells were plated and transfected as described above. Forty-eight hours after transfection, they were washed twice with PBS, detached from the plates with PBS-5mM EDTA/5mM EGTA (as described by VILAIN *et al*., abovementioned, 2001) and pelleted 5 min at 13000xg at 4°C. The pellet was then suspended in 100µL Laemmli buffer, submitted to three cycles of freeze-thaw, boiled and spun 15 min at 13000xg at 4°C. Proteins were quantified on the supernatant with the DC Protein Assay (BIORAD). Fifteen µg of total proteins were loaded on a 10% SDS-PAGE and electroblotted onto a HYBOND ECL membrane (AMERSHAM BIOSCIENCE). Membranes were blocked with 5% non fat dry milk in PBS-TWEEN®. Then, the blot was probed overnight at 4°C with a 1:1000 dilution of anti-FLAG antibody M2 (SIGMA-ALDRICH), with 1:2000 dilution of anti-PAX8 antibody (given by G. VASSART) or with 1:1000 anti-TTF1 antibody (DAKO-CYTOMATION). After anti-PAX8 antibody, horseradish peroxidase-conjugated swine anti-rabbit antibody (DAKO-CYTOMATION) and after anti-Flag and anti-TTF1 antibodies, horseradish peroxidase-conjugated sheep anti-mouse antibody was used as second antibody. Bound antibodies were revealed with a chemiluminescence Kit (AMERSHAM BIOSCIENCE).

### Immunocytochemistry

For immunocytochemistry, cells were plated and transfected as described above. Forty-eight hours after transfection, cells were washed, fixed with acetone/methanol (5/95, v/v) during 10 minutes and permeabilised with PBS-Triton 0,1% during 5 minutes. Endogeneous peroxidase activity was blocked with 3% H₂O₂ during 10 minutes. After washing, nonspecific labeling with Universal blocking reagent (BIOGENEX) was blocked. Cells were then incubated with 1:500 dilution of anti-FLAG antibody M2 or with 1:400 dilution of anti-PHOX2B antibody (given by S. LYONNET) during one hour at room temperature. Controls were performed by omiting the primary antibody. After the anti-flag antibody, Multilink antibody (BIOGENEX) and after anti-PHOX2B, horseradish peroxidase-conjugated anti-goat was used as secondary antibody. Finally, the immunoreactive products were visualized using the DAB reagent (BIOGENEX).

### 2) Population study

For the case-control study, the alanine tracts of the *FOXE1* gene in 115 patients with TD and in 129 French Caucasian controls have been sequenced. The results established the existence of six different alleles of FOXE1 disclosed in the following table 1, which alleles correspond to different polyalanine tract lengths depending of a polymorphic GCX repeat in the FOXE1 sequence.

**Table 1**

| Allele | PCR fragment | Encoded polypeptide | Size of polyalanine tract |
|---|---|---|---|
| 1 | SEQ ID No. 7 | SEQ ID No. 13 | 11 |
| 2 | SEQ ID No 8 | SEQ ID No 14 | 12 |
| 3 | SEQ ID No 9 | SEQ ID No 15 | 14 |
| 4 | SEQ ID No 10 | SEQ ID No 16 | 16 |
| 5 | SEQ ID No 11 | SEQ ID No 17 | 17 |
| 6 | SEQ ID No 12 | SEQ ID No 18 | 19 |

Said six different alleles lead to ten genotypes disclosed in the following Table 2.

**Table 2: genotypes of patients and controls (according to the length of the polyalanine tract of FOXE1)**

| genotypes | Patients | | | | | | | | Controls | |
|---|---|---|---|---|---|---|---|---|---|---|
| | Total | | Athyreosis | | Ectopy | | Hypoplasia/ Hemiagenesis | | | |
| | Nb | f(%) | Nb | f(%) | Nb | f(%) | Nb | f(%) | Nb | f (%) |
| 11/14 | 1 | 0.9 | 1 | 2.9 | 0 | 0 | 0 | 0 | 0 | 0 |
| 12/14 | 1 | 0.9 | 1 | 2.9 | 0 | 0 | 0 | 0 | 0 | 0 |
| 12/16 | 2 | 1.7 | 1 | 2.9 | 0 | 0 | 1 | 6.3 | 0 | 0 |
| 14/14 | 73 | 63.5 | 18 | 51.4 | 46 | 71.9 | 9 | 56.3 | 58 | 45 |
| 14/16 | 28 | 24.3 | 10 | 28.6 | 15 | 23.4 | 3 | 18.8 | 51 | 39.5 |
| 16/16 | 5 | 4.3 | 3 | 8.6 | 0 | 0 | 2 | 12.5 | 17 | 13.2 |
| 14117 | 3 | 2.6 | 0 | 0 | 2 | 3.1 | 1 | 6.3 | 1 | 0.8 |
| 14119 | 1 | 0.9 | 1 | 2.9 | 0 | 0 | 0 | 0 | 0 | 0 |
| 16/17 | 0 | 0 | 0 | 0 | 0 | 0 | 0 | 0 | 2 | 1.6 |
| 17/19 | 1 | 0.9 | 0 | 0 | 1 | 1.6 | 0 | 0 | 0 | 0 |
| Total | 115 | 100 | 35 | 100 | 64 | 100 | 16 | 100 | 129 | 100 |

The results shows that the alleles encoding 14- and 16-alanine tracts were present in the vast majority of the subjects : there were found in 95.7% of the alleles of patients and in 98.8% of control alleles. Due to the small number of rare alleles (11, 12, 17, 19 alanines identified in only 4.3% of patients and 1.2% of controls) only the 14 and 16 alanine tracts were used. The longer tracts (17 and 19 alanines) and the shorter ones (11 and 12 alanines) were not *de novo* expansions and were present in one of the unaffected parents of those cases.

The statistical analysis of the genotype distribution in patients and controls is disclosed in the following Table 3.

**Table 3: comparison of genotype distribution in patients and controls**

| Genotypes | Patients | | Controls | | OR | 95% Cl | *P* |
|---|---|---|---|---|---|---|---|
| | Nb | f | Nb | f | | | |
| 14/14 | 73 | 63.5 | 58 | 45 | 1 | | |
| 14/16* | 28 | 24.3 | 51 | 39.5 | 0.44 | [0.24-0.81] | 0.004 |
| 16/16* | 5 | 4.3 | 17 | 13.2 | 0.23 | [0.07-0.73] | 0.004 |
| 16/16 and 14/16 | 33 | 28.7 | 68 | 52.7 | 0.39 | [0.22-0.68] | 0.0005 |
| Other | 9 | 7.8 | 3 | 2.3 | | | |
| Total | 115 | 100 | 129 | 100 | | | |

The results show that the distribution of the 14/14, 14/16 and 16/16 genotypes was significantly different in cases and controls (χ² = 17.2, dof3, P = 0.0006; Table 3). The 14/14 genotype was the most frequent both in cases (63.5%) and in controls (45.0%) whereas the 14/16 and 16/16 were less frequent among patients (24.3% in patients *νs*. 39.5% in controls and 4.3% *vs*. 13.2% respectively). As the 14/14 genotype and the 14 allele were the most frequent in the control group, they were used as reference for the OR calculations.

The statistical analysis revealed an OR for the 16/16 and 16/14 genotypes versus the 14/14 genotype at 0.39 (95% CI = 0.22-0.68, P = 0.0005), suggesting that the presence of a 16 alanine tract could protect against the occurrence of TD in comparison to the 14/14 genotype (see. Table 3). An allele dose effect was even observed: the risk of TD decreased with the number of copies of the allele 16 in the genotype. Indeed, an OR of 0.44 was observed for the 14/16 genotype *νs*. 14/14 (95% CI = 0.24-0.81, P = 0.004) whereas an OR of 0.23 was found for the 16/16 genotype *vs.* 14/14 (95% CI = 0.07-0.73, P = 0.004) for TD occurrence.

Furthermore, because the occurrence of TD has a striking sexual dimorphism (two to three affected females for one affected male) (CASTANET *et al*., abovementioned, 2001; EUGENE et al., J Clin. Endocrinol. Metab., vol.90, p:2696-700, 2005), we conducted the same analysis in males and females separately. The results remain similar both in males (OR=0.37 for the 16/16 and 16/14 versus the 14/14 genotype 95% CI=0.14 - 0.96, P = 0.024) and in females (OR=0.40 ; 95% CI = 0.19 - 0.85, P = 0.009).

Additionnally, since it is uncertain whether athyreosis and ectopy result from the same or from different mechanisms (GAGNE et al., J. Clin. Endocrinol. Metab., vol.83, p:1771-5, 1998), the analysis was performed in the two subgroups separately.

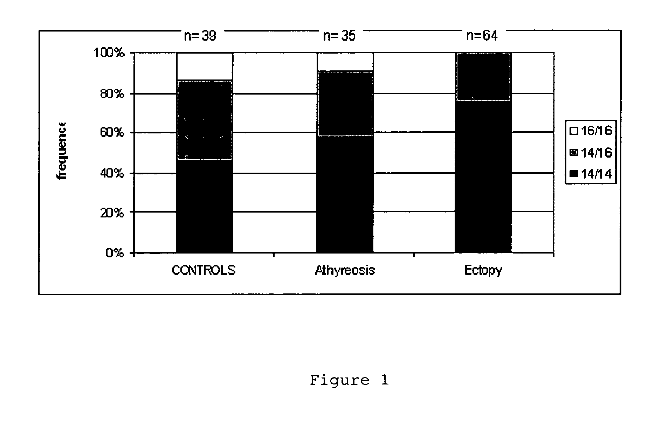

The figure 1 shows the frequency of genotypes (14/14, 14/16 and 16/16) in control group and in the two athyreosis and ectopy groups of patients.

The Odds ratio (OR) of genotypes polyalanine in ectopy or athyreosis groups versus controls is described in the following table 4 and table 5 respectively.

**Table 4**

| Genotypes | Ectopy group | | Controls | | OR | 95% Cl | P |
|---|---|---|---|---|---|---|---|
| | Nb | f | Nb | f | | | |
| 16/16 and 16/14 | 15 | 23.4 | 68 | 52.7 | 0.28 | 0.13-0.58 | 0.00015 |
| 14/14 | 46 | 71.9 | 58 | 45.0 | 1 | | |
| others | 3 | 4.7 | 3 | 2.3 | | | |
| Total | 64 | 100 | 129 | 100 | | | |

**Table 5**

| Genotypes | athyreosis group | | Controls | | OR | 95% Cl | P |
|---|---|---|---|---|---|---|---|
| | Nb | f | Nb | f | | | |
| 16/16 and 16/14 | 13 | 37.2 | 68 | 52.7 | 0.62 | 0.26-1.46 | 0.23 |
| 14/14 | 18 | 51.4 58 | | 45.0 | 1 | | |
| others | 4 | 11.4 | 3 | 2.3 | | | |
| Total | 35 | 100 | 129 | 100 | | | |

The results established that patients with ectopy carried allele 16 significantly less often than patients with athyreosis (14.8% *vs.* 30.0%; P = 0.016). Moreover, the proportion of the 16/16 and 16/14 genotypes was significantly lower in the subgroup of patients affected by ectopy than in the control group (23.4% *vs.* 52.7%, P= 0.0002) (see. Tables 4 and 5, and figure 1), while there was no differences between patients affected by athyreosis and the controls (37.2% *vs.* 52.7 %, P=0.2298). The OR of patients affected by ectopy for the 16/16 and 16/14 genotypes versus the 14/14 genotype was 0.28 (95%CI = 0.13-0.58, P = 0,00015). By contrast, in the group of patients affected by athyreosis, this association was not significant (OR = 0.62; 95%CI = 0.26-1.46, P =0.23). The association observed in the whole cohort was stronger when only the subgroup of patients affected by ectopy was considered.

To confirm the results of the case-control association study, the transmission disequilibrium test (TDT) was used. For this analysis, only parents bearing the 14 and/or 16 alleles with at least one parent being informative (i.e heterozygous 14/16) were considered, which allowed to select 39 trios. Among them, 26 parents transmitted allele 14 to their affected child while 13 parents transmitted allele 16 (χ² = 4.3, P = 0.038).

Finally, the results strongly suggest that the length of the alanine stretch within FOXE1 modulates genetic susceptibility to TD. Indeed, patients affected by TD present a significantly lower proportion of the 16/16 and 16/14 genotypes compared to controls (28.7% *vs* 52.7%). The OR at 0.39 strongly suggests that the presence of 16 alanine either at heterozygote state (i.e 14/16 genotype) or homozygote state (i.e 16/16 genotype) significantly protects from the occurrence of TD in comparison with the 14/14 genotype. Thus, TD is associated with the more common variant (14) suggesting that the less common variant (16) may offer protection against developing the disease (SLADEK et al., Nature, vol.445(7130), p:881-5, 2007; FREIMER et al., Nature, vol.445(7130), p:828-30, 2007).

It may be surprising that the most common genotype in the cohort of controls as well as in affected cases confers risk but this has been previously described for some other gene polymorphisms and diseases (MALIK et al., Proc. Natl. Acad. Sci. U S A, vol.102, p:12183-12188, 2005). Additionally, the fact that protection against the occurrence of TD was more significant for ectopy might be in accordance with the role of Foxel in thyroid development. Indeed, knockout mice data suggested that Foxe1 could control migration of the follicular cells from the foramen caecum of the tongue to the neck and their terminal differentiation (DE FELICE and DI LAURO, abovementioned, 2004).

### 3) Functional study

Alanine-tract expansions in transcription factors have been implicated as a cause of some human diseases. One example is congenital central hypoventilation syndrome (OMIM 209880) where expansions of the alanine tract in *PHOX2B,* a paired-type homeobox transcription factor, have been directly linked to the disease (AMIEL et al., Nat. Genet., vol.33, p:459-461, 2003).

As the alleles encoding 14- and 16-alanine tracts were present in the vast majority of patients and controls, the functional effects of the 14 and 16 residues only were investigated by transiently transfecting eukaryotic cells co-expressing the luciferase gene directed by the human thyroglobulin gene promoter.

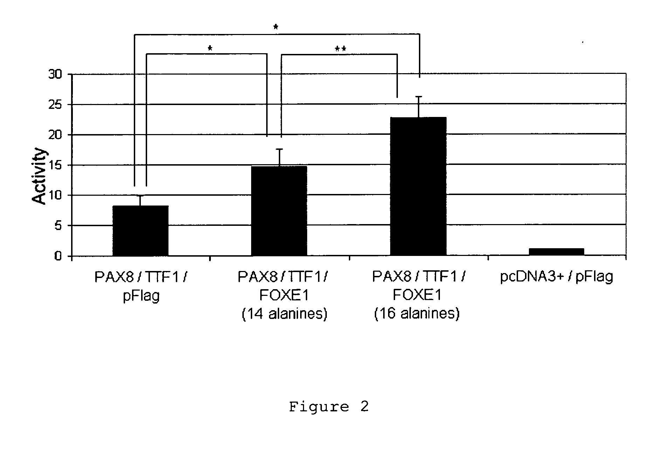

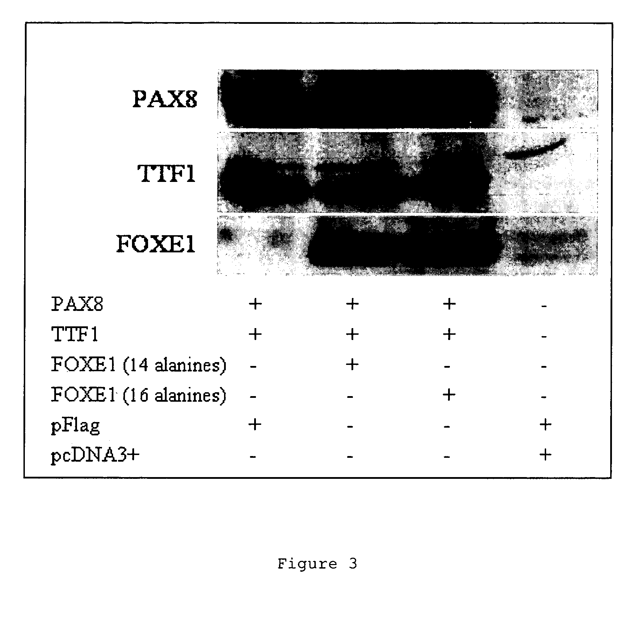

The figure 2 shows the effects of the transfection in 293A cells of PAX8 and TTF1 with or without FOXE1 (14 or 16 alanines) on transcriptional activity. The data represent the mean ± SD in 7 assays (** p<0.003 (Mann-Whitney test); * p<0.002 (Mann-Whitney test)). As a control, the figure 3 shows protein expression of PAX8, TTF1, and FOXE1 (14 or 16 alanines) in these distinct transfection experiments as assessed by Western blot.

The results show that the plasmid coding for FOXE1 with 16 alanines induced a stronger transactivation than with 14 alanines in the context of synergy with constant amounts of PAX8 and TTF1 (activity over baseline 22.8 ± 3.4 SD *vs* 14.7 ± 2 SD, P < 0.003) (*see.* figures. 2 and 3). Thus, the results established that the ability of FOXE1 to transactivate a target gene was significantly modified by the length of the alanine tracts.

Furthermore, to investigate whether the cellular localisation of FOXE1 dependied on the length of the alanine stretch, we performed immunocytochemistry.The results have shown that similar amounts of FOXE1 protein were detected in the nucleus with constructs containing 14 or 16 alanines. In contrast, as an internal control and as previously described (TROCHET *et al.,* abovementioned, 2005), the results have shown that the PHOX2B transcription factor aggregated in the cytoplasm when containing 33 alanines instead of 20 alanines.

Finally, these results demonstrates for the first time that the length of the alanine tract within FOXE1 modified the transactivation ability in the presence of TTF1 and PAX8, a combination of transcription factors whose simultaneous expression is unique to thyroid follicular cells (SURA-TRUEBA *et al.,.* abovementioned, 2005). Previous studies did not find difference between polyA tract length varying from 11 to 14 residues for luciferase expression. The authors concluded that the polymorphism in FOXE1 polyA tract was unlikely responsible for TD (HISHINUMA *et al.,* abovementioned, 2001).

In conclusion, the data strongly suggests that the mechanism whereby the 16 alanine tract of the FOXE1 confers protection towards the occurrence of TD and in a more marked way for ectopy *νs*. the 14 alanine tract acts through increased transcriptional activity. These new findings are in favour of a role of FOXE1 on the migration of the thyroid gland through its polyA tracts. Note that these two phenomenoms (i.e., migration and differentiation) are considered to be mutually exclusive so that adequate control of the genes involved in terminal differentiation during the migration phase is crucial (DE FELICE and DI LAURO, abovementioned, 2004). In summary, our data strongly point to a role of the FOXE1 polyA tract length in TD, demonstrating a new mode of action of an alanine containing transcription factor in relation to disease. Nevertheless, multifactorial origin of TD is now recognised (AMENDOLA et al., Endocrinology, vol.146, p:5038-4, 2005) and variations of polyA tract could be regarded as a modulator of genetic susceptibility for TD in such a polygenic model.

### Example 2: FOXE1 polyalanine repeat length and thyroid hormones synthesis during pregnancy

### 1) Materials and Methods

### Subjects and samples

110 unrelated pregnant women, which are all French Caucasian affected with TD were included in the study.

Blood samples are taken from these women at regular interval during pregnancy.

Genomic DNA was extracted from peripheral blood using standard procedures.

### Alanine tract sequencing

Alanine sequencing is realized as in example 1.

### Thyroid hormones and TSH levels

T3, T4 and TSH levels are measured by Chemiluminescence Immuno Assay on ACS:180 (Siemens Medical Solutions Diagnostics) according to the manufacturer's instruction.

### Statistical analysis

The statistical analysis is realized as in example 1 for determining whether the allele of *FOXE1* comprising a polyalanine repeat with 16 successive alanines results in a greatest thyroid hormones and TSH synthesis during pregnancy *vs*. the allele comprising 14 successive alanines.

## Claims

1. A method for diagnosis an increased likelihood of developing a thyroid dysgenesis (TD) for an individual, wherein said method comprises determining the length of the polyalanine repeat of the protein encoded by at least one allele of the FOXE1 gene in a tissue sample obtained from said individual.

2. The method of claim 1, wherein said thyroid dysgenesis comprises ectopic sublingual thyroid, athyreosis, and thyroid hemiagenesis, preferably ectopic sublingual thyroid.

3. The method of any one of claims 1 or 2, wherein the length of the polyalanine repeat corresponds to at least ten successive alanine amino acids, preferably to 11, 12, 14, 16, 17, or 19 successive alanines amino acids.

4. The method of any one of claims 1 to 3, wherein said individual is a human.

5. The method of claim 1, wherein said individual is a female, preferably a pregnant female.

6. The method of claim 5, wherein said method is for diagnosis an increased likelihood of neuropsychological development retardation and/or of developing a thyroid dysgenesis for the foetus during the pregnancy of said female.

7. The method of any one of claims 1 to 6, wherein the protein encoded by at least one allele of the human FOXE 1 gene has the sequence SEQ ID NO: 1.

8. The method of any one of claims 1 to 7, wherein the protein encoded by at least one allele of the human FOXE 1 gene is encoded by the sequence SEQ ID NO:2.

9. The method of any one of claims 1 to 8, wherein the length of the polyalanine repeat of the protein encoded by at least one allele of the FOXE1 gene is determined with a DNA sequencing protocol or with a method involving a polymerase chain reaction using at least one oligonucleotide complementary to the sequence of FOXE1 gene or to the sequence of FOXE1 mRNA, preferably at least one oligonucleotide selected in the group comprising SEQ ID NO:3 and SEQ ID NO:4.

10. A kit comprising at least one nucleic acid probe or oligonucleotide which can be used in a method as defined in any one of claims 1 to 9 for determining the length of the polyalanine repeat of the protein encoded by at least one allele of the FOXE1 gene in a tissue sample obtained from an individual.

11. The kit of claim 10, wherein said kit comprises at least one PCR primer selected in the group comprising SEQ ID NO:3 and SEQ ID NO:4.

12. Use of the kit as defined in any one of claims 10 or 11 for the diagnosis of an increased likelihood of developing a thyroid dysgenesis (TD) in an individual.

13. The use of claim 12, wherein said individual is a female and wherein said kit is for diagnosis an increased likelihood of neuropsychological development retardation and/or of developing a thyroid dysgenesis for the foetus during the pregnancy of said female.


SEQUENCE LISTING

<110> INSTITUT NATIONAL DE LA SANTE ET DE LA RECHERCHE MEDICALE
      (INSERM)
      POLAK, Michel
      CASTANET, Mireille
      CARRE, Aurore

<120> Polymorphic length of foxe1 alanine stretch and genetic
      susceptibility to thyroid dysgenesis

<130> D25372

<160> 18

<170> PatentIn version 3.3


<210> 1
<211> 369
<212> PRT
<213> Homo sapiens
<220>
<221> MISC_FEATURE
<222> (175)..(175)
<223> Xaa= (A)n with n= 0, 1, 2, 5, 6, or 8

<400> 1

Met Thr Ala Glu Ser Gly Pro Pro Pro Pro Gln Pro Glu Val Leu Ala
1               5                   10                  15
Thr Val Lys Glu Glu Arg Gly Glu Thr Ala Ala Gly Ala Gly Val Pro
            20                  25                  30
Gly Glu Ala Thr Gly Arg Gly Ala Gly Gly Arg Arg Arg Lys Arg Pro
        35                  40                  45
Leu Gln Arg Gly Lys Pro Pro Tyr Ser Tyr Ile Ala Leu Ile Ala Met
    50                  55                  60
Ala Ile Ala His Ala Pro Glu Arg Arg Leu Thr Leu Gly Gly Ile Tyr
65                  70                  75                  80
Lys Phe Ile Thr Glu Arg Phe Pro Phe Tyr Arg Asp Asn Pro Lys Lys
                85                  90                  95
Trp Gln Asn Ser Ile Arg His Asn Leu Thr Leu Asn Asp Cys Phe Leu
            100                 105                 110
Lys Ile Pro Arg Glu Ala Gly Arg Pro Gly Lys Gly Asn Tyr Trp Ala
        115                 120                 125
Leu Asp Pro Asn Ala Glu Asp Met Phe Glu Ser Gly Ser Phe Leu Arg
    130                 135                 140
Arg Arg Lys Arg Phe Lys Arg Ser Asp Leu Ser Thr Tyr Pro Ala Tyr
145                 150                 155                 160
Met His Asp Ala Ala Ala Ala Ala Ala Ala Ala Ala Ala Ala Xaa Ile
                165                 170                 175
Phe Pro Gly Ala Val Pro Ala Ala Arg Pro Pro Tyr Pro Gly Ala Val
            180                 185                 190
Tyr Ala Gly Tyr Ala Pro Pro Ser Leu Ala Ala Pro Pro Pro Val Tyr
        195                 200                 205
Tyr Pro Ala Ala Ser Pro Gly Pro Cys Arg Val Phe Gly Leu Val Pro
    210                 215                 220
Glu Arg Pro Leu Ser Pro Glu Leu Gly Pro Ala Pro Ser Gly Pro Gly
225                 230                 235                 240
Gly Ser Cys Ala Phe Ala Ser Ala Gly Ala Pro Ala Thr Thr Thr Gly
                245                 250                 255

Tyr Gln Pro Ala Gly Cys Thr Gly Ala Arg Pro Ala Asn Pro Ser Ala
            260                 265                 270
Tyr Ala Ala Ala Tyr Ala Gly Pro Asp Gly Ala Tyr Pro Gln Gly Ala
        275                 280                 285
Gly Ser Ala Ile Phe Ala Ala Ala Gly Arg Leu Ala Gly Pro Ala Ser
    290                 295                 300
Pro Pro Ala Gly Gly Ser Ser Gly Gly Val Glu Thr Thr Val Asp Phe
305                 310                 315                 320
Tyr Gly Arg Thr Ser Pro Gly Gln Phe Gly Ala Leu Gly Ala Cys Tyr
                325                 330                 335
Asn Pro Gly Gly Gln Leu Gly Gly Ala Ser Ala Gly Ala Tyr His Ala
            340                 345                 350
Arg His Ala Ala Ala Tyr Pro Gly Gly Ile Asp Arg Phe Val Ser Ala
        355                 360                 365
Met

<210> 2
<211> 1107
<212> DNA
<213> Homo sapiens

<220>
<221> misc_feature
<222> (523)..(525)
<223> nnn= (GCC)n wih n= 0, 1, 2, 5, 6, or 8

<400> 2

atgactgccg agagcgggcc gccgccgccg cagccggagg tgctggctac cgtgaaggaa      60
gagcgcggcg agacggcagc aggggccggg gtcccagggg aggccacggg ccgcggggcg     120
ggcgggcggc gccgcaagcg ccccctgcag cgcgggaagc cgccctacag ctacatcgcg     180
ctcatcgcca tggccatcgc gcacgcgccc gagcgccgcc tcacgctggg cggcatctac     240
aagttcatca ccgagcgctt ccccttctac cgcgacaacc ccaaaaagtg gcagaacagc     300
atccgccaca acctcacact caacgactgc ttcctcaaga tcccgcgcga ggccggccgc     360
ccgggtaagg gcaactactg ggcgcttgac cccaacgcgg aggacatgtt cgagagcggc     420
agcttcctgc gccgccgcaa gcgcttcaag cgctcggacc tctccaccta cccggcttac     480
atgcacgacg cggcggctgc cgcagccgcc gccgccgccg ccnnnatctt cccaggcgcg     540
gtgcccgccg cgcgcccccc ctacccgggc gccgtctatg caggctacgc gccgccgtcg     600
ctggccgcgc cgcctccagt ctactacccc gcggcgtcgc ccggcccttg ccgcgtcttc     660
ggcctggttc ctgagcggcc gctcagccca gagctggggc ccgcaccgtc ggggcccggc     720
ggctcttgcg cctttgcctc cgccggcgcc cccgctacca ccaccggcta ccagcccgca     780
ggctgcaccg gggcccggcc ggccaacccc tccgcctatg cggctgccta cgcgggcccc     840
gacggcgcgt acccgcaggg cgccggcagt gcgatctttg ccgctgctgg ccgcctggcg     900
ggacccgctt cgcccccagc gggcggcagc agtggcggcg tggagaccac ggtggacttc     960
tacgggcgca cgtcgcccgg ccagttcgga gcgctgggag cctgctacaa ccctggcggg    1020
cagctcggag gggccagtgc aggcgcctac catgctcgcc atgctgccgc ttatcccggt    1080
gggatagatc ggttcgtgtc cgccatgtga                                     1110

<210> 3
<211> 19
<212> DNA
<213> artificial sequence

<220>
<223> Primer

<400> 3

gcggaggaca tgttcgaga                                                    19

<210> 4
<211> 20
<212> DNA
<213> artificial sequence

<220>
<223> Primer

<400> 4

cgcggggtag tagactggag                                                   20

<210> 5
<211> 24
<212> DNA
<213> artificial sequence

<220>
<223> Primer

<400> 5

cgccgccgcc gcagcaatct tccc                                              24

<210> 6
<211> 24
<212> DNA
<213> artificial sequence

<220>
<223> Primer

<400> 6

gggaagattg ctgcggcggc ggcg                                              24

<210> 7
<211> 239
<212> DNA
<213> artificial sequence

<220>
<223> 11A FOXE1 PCR fragment

<400> 7

gcggaggaca tgttcgagag cggcagcttc ctgcgccgcc gcaagcgctt caagcgctcg      60
gacctctcca cctacccggc ttacatgcac gacgcggcgg ctgccgcagc cgccgccgcc     120
gccgccatct tcccaggcgc ggtgcccgcc gcgcgccccc cctacccggg cgccgtctat     180
gcaggctacg cgccgccgtc gctggccgcg ccgcctccag tctactaccc cgcggcgtc      239

<210> 8
<211> 242
<212> DNA

<213> artificial sequence

<220>
<223> 12A FOXE1 PCR fragment

<400> 8

gcggaggaca tgttcgagag cggcagcttc ctgcgccgcc gcaagcgctt caagcgctcg 60
gacctctcca cctacccggc ttacatgcac gacgcggcgg ctgccgcagc cgccgccgcc 120
gccgccgcca tcttcccagg cgcggtgccc gccgcgcgcc ccccctaccc gggcgccgtc 180
tatgcaggct acgcgccgcc gtcgctggcc gcgccgcctc cagtctacta ccccgcggcg 240
tc 242


<210> 9
<211> 248
<212> DNA
<213> artificial sequence

<220>
<223> 14A FOXE1 PCR fragment

<400> 9

gcggaggaca tgttcgagag cggcagcttc ctgcgccgcc gcaagcgctt caagcgctcg 60
gacctctcca cctacccggc ttacatgcac gacgcggcgg ctgccgcagc cgccgccgcc 120
gccgccgccg ccgccatctt cccaggcgcg gtgcccgccg cgcgcccccc ctacccgggc 180
gccgtctatg caggctacgc gccgccgtcg ctggccgcgc cgcctccagt ctactacccc 240
gcggcgtc 248


<210> 10
<211> 254
<212> DNA
<213> artificial sequence

<220>
<223> 16A FOXE1 PCR fragment

<400> 10

gcggaggaca tgttcgagag cggcagcttc ctgcgccgcc gcaagcgctt caagcgctcg 60
gacctctcca cctacccggc ttacatgcac gacgcggcgg ctgccgcagc cgccgccgcc 120
gccgccgccg ccgccgccgc catcttccca ggcgcggtgc ccgccgcgcg ccccccctac 180
ccgggcgccg tctatgcagg ctacgcgccg ccgtcgctgg ccgcgccgcc tccagtctac 240
taccccgcgg cgtc 254


<210> 11
<211> 257
<212> DNA
<213> artificial sequence

<220>
<223> 17A FOXE1 PCR fragment

<400> 11

gcggaggaca tgttcgagag cggcagcttc ctgcgccgcc gcaagcgctt caagcgctcg 60
gacctctcca cctacccggc ttacatgcac gacgcggcgg ctgccgcagc cgccgccgcc 120
gccgccgccg ccgccgccgc cgccatcttc ccaggcgcgg tgcccgccgc gcgccccccc 180

tacccgggcg ccgtctatgc aggctacgcg ccgccgtcgc tggccgcgcc gcctccagtc     240
tactaccccg cggcgtc                                                    257

<210> 12
<211> 263
<212> DNA
<213> artificial sequence

<220>
<223> 19A FOXE1 PCR fragment

<400> 12
   gcggaggaca tgttcgagag cggcagcttc ctgcgccgcc gcaagcgctt caagcgctcg      60
   gacctctcca cctacccggc ttacatgcac gacgcggcgg ctgccgcagc cgccgccgcc     120
   gccgccgccg ccgccgccgc cgccgccgcc atcttcccag gcgcggtgcc cgccgcgcgc     180
   cccccctacc cgggcgccgt ctatgcaggc tacgcgccgc cgtcgctggc cgcgccgcct     240
   ccagtctact accccgcggc gtc                                             263


<210> 13
<211> 79
<212> PRT
<213> artificial sequence

<220>
<223> Polypeptide encoded by 11A FOXE1 PCR fragment

<400> 13

Ala Glu Asp Met Phe Glu Ser Gly Ser Phe Leu Arg Arg Arg Lys Arg
1               5                   10                  15
Phe Lys Arg Ser Asp Leu Ser Thr Tyr Pro Ala Tyr Met His Asp Ala
            20                  25                  30
Ala Ala Ala Ala Ala Ala Ala Ala Ala Ala Ile Phe Pro Gly Ala Val
        35                  40                  45
Pro Ala Ala Arg Pro Pro Tyr Pro Gly Ala Val Tyr Ala Gly Tyr Ala
    50                  55                  60
Pro Pro Ser Leu Ala Ala Pro Pro Pro Val Tyr Tyr Pro Ala Ala
65                  70                  75


<210> 14
<211> 80
<212> PRT
<213> artificial sequence

<220>
<223> Polypeptide encoded by 12A FOXE1 PCR fragment

<400> 14

Ala Glu Asp Met Phe Glu Ser Gly Ser Phe Leu Arg Arg Arg Lys Arg
1               5                   10                  15
Phe Lys Arg Ser Asp Leu Ser Thr Tyr Pro Ala Tyr Met His Asp Ala
            20                  25                  30
Ala Ala Ala Ala Ala Ala Ala Ala Ala Ala Ala Ile Phe Pro Gly Ala
        35                  40                  45
Val Pro Ala Ala Arg Pro Pro Tyr Pro Gly Ala Val Tyr Ala Gly Tyr
    50                  55                  60
Ala Pro Pro Ser Leu Ala Ala Pro Pro Pro Val Tyr Tyr Pro Ala Ala

65                  70                  75                  80


<210> 15
<211> 82
<212> PRT
<213> artificial sequence

<220>
<223> Polypeptide encoded by 14A FOXE1 PCR fragment

<400> 15

Ala Glu Asp Met Phe Glu Ser Gly Ser Phe Leu Arg Arg Arg Lys Arg
1               5                   10                  15
Phe Lys Arg Ser Asp Leu Ser Thr Tyr Pro Ala Tyr Met His Asp Ala
            20                  25                  30
Ala Ala Ala Ala Ala Ala Ala Ala Ala Ala Ala Ala Ala Ile Phe Pro
        35                  40                  45
Gly Ala Val Pro Ala Ala Arg Pro Pro Tyr Pro Gly Ala Val Tyr Ala
    50                  55                  60
Gly Tyr Ala Pro Pro Ser Leu Ala Ala Pro Pro Pro Val Tyr Tyr Pro
65                  70                  75                  80
Ala Ala



<210> 16
<211> 84
<212> PRT
<213> artificial sequence

<220>
<223> Polypeptide encoded by 16A FOXE1 PCR fragment

<400> 16

Ala Glu Asp Met Phe Glu Ser Gly Ser Phe Leu Arg Arg Arg Lys Arg
1               5                   10                  15
Phe Lys Arg Ser Asp Leu Ser Thr Tyr Pro Ala Tyr Met His Asp Ala
            20                  25                  30
Ala Ala Ala Ala Ala Ala Ala Ala Ala Ala Ala Ala Ala Ala Ala Ile
        35                  40                  45
Phe Pro Gly Ala Val Pro Ala Ala Arg Pro Pro Tyr Pro Gly Ala Val
    50                  55                  60
Tyr Ala Gly Tyr Ala Pro Pro Ser Leu Ala Ala Pro Pro Pro Val Tyr
65                  70                  75                  80
Tyr Pro Ala Ala



<210> 17
<211> 85
<212> PRT
<213> artificial sequence

<220>
<223> Polypeptide encoded by 17A FOXE1 PCR fragment

<400> 17

Ala Glu Asp Met Phe Glu Ser Gly Ser Phe Leu Arg Arg Arg Lys Arg
1               5                   10                  15
Phe Lys Arg Ser Asp Leu Ser Thr Tyr Pro Ala Tyr Met His Asp Ala
            20                  25                  30
Ala Ala Ala Ala Ala Ala Ala Ala Ala Ala Ala Ala Ala Ala Ala Ala
        35                  40                  45
Ile Phe Pro Gly Ala Val Pro Ala Ala Arg Pro Pro Tyr Pro Gly Ala
    50                  55                  60
Val Tyr Ala Gly Tyr Ala Pro Pro Ser Leu Ala Ala Pro Pro Pro Val
65                  70                  75                  80
Tyr Tyr Pro Ala Ala
                85

<210> 18
<211> 87
<212> PRT
<213> artificial sequence

<220>
<223> Polypeptide encoded by 19A FOXE1 PCR fragment

<400> 18

Ala Glu Asp Met Phe Glu Ser Gly Ser Phe Leu Arg Arg Arg Lys Arg
1               5                   10                  15
Phe Lys Arg Ser Asp Leu Ser Thr Tyr Pro Ala Tyr Met His Asp Ala
            20                  25                  30
Ala Ala Ala Ala Ala Ala Ala Ala Ala Ala Ala Ala Ala Ala Ala Ala
        35                  40                  45
Ala Ala Ile Phe Pro Gly Ala Val Pro Ala Ala Arg Pro Pro Tyr Pro
    50                  55                  60
Gly Ala Val Tyr Ala Gly Tyr Ala Pro Pro Ser Leu Ala Ala Pro Pro
65                  70                  75                  80
Pro Val Tyr Tyr Pro Ala Ala
                85